# EUROPEAN PATENT APPLICATION

(11) **EP 4 108 226 A1**
(43) Date of publication of application: **28.12.2022**
(21) Application number: 21180766.4
(22) Date of filing: 22.06.2021
(51) Int. Cl.: A61K 8/23, A61K 8/40, A61K 8/67, A61Q 3/00

(54) **NAIL GLUE COMPOSITION**

(71) Applicant: Chemence Limited, London WC2B 4AS (GB)
(72) Inventor: Russell, Michael, Corby, NN17 4XD (GB)
(74) Representative: Sanger, Phillip Simon

(57) **Abstract**

A nail glue composition comprising:
at least one cyanoacrylate of the formula:

CH₂=C(CN)COOR

wherein R is ethyl, methoxyethyl, ethoxyethyl, *n*-butyl or octyl or mixtures thereof;
an anionic stabiliser; and
an antioxidant stabiliser comprising at least one stabiliser compound selected from the group consisting of:
α -tocopherol, *β*-tocopherol, γ-tocopherol, δ-tocopherol, α-tocotrienol, *β-*tocotrienol, γ-tocotrienol and *δ*-tocotrienol; and
derivatives thereof;

in which the at least one stabiliser compound is in an amount of at least 0.02wt% of the nail glue composition.

## Description

### Technical Field

The present invention relates to a nail glue composition. More specifically, the present invention relates to a composition for a nail glue that negates the use of various phenolic stabilisers/antioxidants such as Butylated Hydroxy Anisole (BHA), Hydroquinone (HQ) and 4-Methoxyphenol (MEHQ).

### Background Art

It is common in the beauty industry to adhere artificial nails onto natural nails (i.e., human fingernails or toenails) using an adhesive. Artificial nails are considered to provide improved appearance and durability over natural nails. Most artificial nail tips are manufactured out of plastic such as acrylonitrile butadiene styrene (ABS), a durable yet flexible material that can be shaped into a smooth surface that resists yellowing.

Typically, such adhesives are cyanoacrylate adhesives. These adhesives, also known as "superglues", are strong and fast-acting.

Most nail glues used to bond artificial nails to natural nails are based on 2-ethyl cyanoacrylate, but other cyanoacrylates have and can be used including n-butyl cyanoacrylate, 2-Octyl cyanoacrylate, 2-Methoxyethyl Cyanoacrylate and 2-Ethoxyethyl 2-Cyanoacrylate.

Nail glue compositions comprise compounds that acts as inhibitors / stabilisers. The purpose of these stabilisers is to improve the shelf life of the product and to prevent premature polymerisation of the monomeric forms of cyanoacrylates.

Phenolic inhibitors / stabilisers are added both during and after the manufacturing process for monomers (especially acrylate monomers) to prevent or slow auto polymerisation by absorbing free radicals, such as the C (Carbon atom with a single free electron) to produce substances unable to induce further polymerisation. They have been, and still are, widely used in the polymer industry but have fallen out of favour when used in cosmetic products for safety reasons.

Previously, the main inhibitor used for cosmetic nail glues was Hydroquinone (HQ). However, in 2002, along with Hydroquinone Methyl Ether (MEHQ), its use in cosmetic nail glues placed on the market in the European Union was limited to 'Professional Use Only' and the maximum permissible level set at 0.02wt% or 200ppm.

The cosmetic nail glue industry looked for alternatives and there were two likely candidates based on effectiveness and cost: Butylated Hydroxy Anisole (BHA) and Butylated Hydroxytoluene (BHT). Of the two, BHA was a better stabiliser and the industry mostly replaced HQ with BHA.

A typical prior art adhesive composition for nail glues comprises a polymerizable adhesive monomer, typically 2-ethyl cyanoacrylate, a viscosity modifier, typically polymethyl methacrylate (PMMA), a free radical stabiliser such as Butylated Hydroxy Anisole (BHA) and suitable anionic stabilisers including Lewis acids such as Sulphur Dioxide, Boron Trifluoride and other similar anionic stabilisers. The anionic stabiliser is added at very low levels, typically 2 ppm to 40 ppm.

More recently, BHA has been classified as a Class 2 carcinogen suspected of causing cancer. Therefore, the applicant has identified that BHA may eventually be banned or its use severely limited in cosmetics in the EU - as with HQ and MEHQ. In the US State of California, BHA must be listed as a carcinogen on any products that contain it (such as nail glues) under Proposition 65, officially known as the Safe Drinking Water and Toxic Enforcement Act of 1986,

What is required is an alternative inhibitor stabiliser that exhibits the same or superior qualities to the prior art (HQ, MEHQ, BHA) but without the health risks.

A prior art nail glue composition is disclosed in US 2019/0298628 A1. The aim of the invention proposed in US'628 is to eliminate ethyl cyanoacrylate from the composition due to its odour, brittleness and purported health implications. In this document, various compositions are disclosed in which a vitamin additive is provided, selected from Vitamin A, Vitamin E and a combination thereof. Each vitamin is added at 0.01wt%. The vitamins are added exclusively as therapeutic agents to improve the care and protection of the user's nails. The vitamins are not added in an amount that would provide any statistically significantstabilising effect. Furthermore, US'628 referstothe addition of "Vitamin E", which means that a combination of the eight compounds discussed below are added.

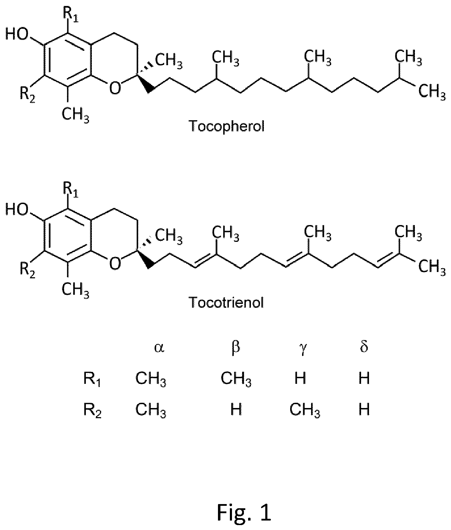

Vitamin E refers to a group of eight different compounds: α-, β-, γ-, and δ-tocopherols and the corresponding four tocotrienols. The α-, β-, γ-, and δ-forms differ with respect to the number and position of methyl groups on the chromanol ring. The α-forms of tocopherol and tocotrienol have three methyl groups at the C5, C7, and C8-positions of the chromanol ring, while the β- and γ-forms have two and the δ-forms have one methyl group as illustrated in Figure 1.

### Summary of Invention

According to a first aspect of the present invention there is provided a nail glue composition comprising:
at least one cyanoacrylate of the formula:

   CH₂=C(CN)COOR
wherein R is ethyl, methoxyethyl, ethoxyethyl, n-butyl or octyl or mixtures thereof;
an anionic stabiliser; and
an antioxidant stabiliser comprising at least one stabiliser compound selected from the group consisting of:
   *α*-tocopherol, *β*-tocopherol, *γ*-tocopherol, *δ*-tocopherol, *α*-tocotrienol, *β*-tocotrienol, *γ*-tocotrienol and *δ*-tocotrienol and derivatives thereof in an amount of at least 0.02wt% of the nail glue composition.

Advantageously, the addition of at least one of the Vitamin E compounds *α-*tocopherol, *β*-tocopherol, *γ*-tocopherol, *δ*-tocopherol, *α*-tocotrienol, *β*-tocotrienol, *γ-*tocotrienol and *δ*-tocotrienol (and / or derivatives thereof) to an α-cyanoacrylate adhesive formulation in the quantities claimed, in full (or partial) replacement of the various phenolic stabilisers that might be used, has the surprising effect of increasing the durability and longevity of the adhesion of the artificial nail on the natural nail together with an increase in the tensile strength of the bond. The composition according to the invention is BHA-, HQ- and MEHQ-free.

The at least one stabiliser compound may be a single compound (such as *α-*tocopherol) or a combination of compounds.

The term "Vitamin E" is used to denote the group of fat-soluble compounds consisting of *α*-tocopherol, *β*-tocopherol, *γ*-tocopherol, *δ*-tocopherol, *α*-tocotrienol, *β-*tocotrienol, *γ*-tocotrienol and *δ*-tocotrienol. Each of these individually is described herein as a "Vitamin E compound"- i.e. a compound of the above group.

It has been discovered that the Vitamin E compounds are effective stabilisers and can individually or in combination be used as a direct replacement for BHA in nail glue formulations.

Preferably the antioxidant stabiliser is present in an amount of at least 0.02wt% of the nail glue composition, more preferably at least 0.05wt%, and most preferably at least 0.2wt% of the nail glue composition.

Preferably, at least one cyanoacrylate is in the range of 85 to 99 wt% of the nail glue composition.

Preferably the antioxidant stabiliser is one of the Vitamin E compounds (i.e. *α-*tocopherol, *β*-tocopherol, *γ*-tocopherol, *δ*-tocopherol, *α*-tocotrienol, *β*-tocotrienol, *γ-*tocotrienol or *δ*-tocotrienol). More preferably, the antioxidant stabiliser is *α*-tocopherol.

Preferably, the Vitamin E compound is selected in isolation. More preferably *α-*tocopherol is provided in isolation (i.e. no other Vitamin E compounds are present). The applicant has discovered that isolation of a single compound (in particular *α*-tocopherol) is more effective.

Therefore according to a second aspect of the present invention there is provided a nail glue composition comprising:
at least one cyanoacrylate of the formula:

   CH₂=C(CN)COOR
wherein R is ethyl, methoxyethyl, ethoxyethyl, n-butyl or octyl or mixtures thereof;
an anionic stabiliser; and
an antioxidant stabiliser comprising at least 0.02wt% of *α*-tocopherol.

Experiments conducted by the applicant indicate that utilising *α*-tocopherol as a stabiliser compound in an amount of at least 0.02wt% of the nail glue composition is highly effective compared to the equivalent amount of other Vitamin E compounds or combinations thereof.

Preferably the composition includes a thickener. More preferably the thickener is polymethyl methacrylate (PMMA). Most preferably the thickener is present in the range of 0.1wt% to 14wt% of the nail glue composition.

Preferably the antioxidant stabiliser is present in the range 0.02wt%to 0.5wt% of the nail glue composition.

The general form of the invention is a mixture of a cyanoacrylate monomer adhesive, acidic stabilisers and a Vitamin E compound of at least 0.02wt%, without the presence of any phenolic stabilisers, applied in a thin layer between an artificial nail and a natural nail.

Preferably, the adhesive should be a low molecular weight aliphatic cyanoacrylate combined with a thickener together with a small amount of the Vitamin E compound and acidic stabilisers.

More preferably, the formula should comprise thickener in the range 2wt%to 15wt% by weight combined with the Vitamin E compound in the range 0.02wt% to 0.5wt% and less than 0.01wt% of acidic stabilisers with the balance comprising the cyanoacrylate monomer.

Still more preferably, the thickener should be polymethyl methacrylate (PMMA) and the monomer should be 2-ethyl cyanoacrylate.

Still more preferably, the formula should comprise *α*-tocopherol in the range 0.1wt% to 0.3wt%, PMMA in the range 4wt% to 6wt% and the balance comprise 2-ethyl cyanoacrylate and acidic stabilisers. The modified adhesive must be compatible with both the natural nail (a-keratin) and the artificial nail material (e.g., acrylonitrile butadiene styrene (ABS)).

### Brief description of the drawings

FIGURE 1 is a chemical structure drawing of various Vitamin E compounds.

### Specific description

The following specific formulation is one example of how the present invention may be carried out:

**Table 1**

| **Formulation 1** | **CAS#** | **Percent w/w** |
|---|---|---|
| Ethyl cyanoacrylate | 7085-85-0 | 95.3 |
| Polymethyl methacrylate (PMMA) | 9011-14-7 | 4.5 |
| *α*-tocopherol | 59-02-9 | 0.2 |
| **TOTAL** | | **100** |

Testing by the inventor has shown that the formulation in Table 1 is unexpectedly superior to nail glues containing traditional phenolic stabilisers in increasing both the longevity and durability of the adhesion of the artificial nail to the natural nail, and in improving the lap shear tensile strength of the nail glue measured by the tensile strength required to break the adhesive bond when applied between two strips of mild steel and similarly between two strips of acrylonitrile butadiene styrene (ABS) plastic.

### Test results - stability

Various amounts of α-tocopherol and BHA were added separately to ethyl 2-cyanaocrylate monomer with no thickener added and only anionic stabiliser. Samples were then placed in a temperature-controlled oven at 82°C for three days and removed and allowed to cool to 20°C. The initial viscosity at 20°C measured in cPs, was then compared with the viscosity after storage for three days at an elevated temperature. The results of the above experiment show that *α*-tocopherol has a similar or greater stabilising effect on ethyl-2-cyanaocrylate monomer when compared to BHA at similar addition levels. Therefore, *α*-tocopherol can be used as a direct replacement for BHA in cyanoacrylate adhesives.

### Test results - longevity

Twenty (adult) participants participated in a longevity test whereby a thin layer of Formulation 1 adhesive was applied to the natural nails of the dominant hand and an artificial nail made from acrylonitrile butadiene styrene (ABS) plastic was adhered to each natural nail by pressing down firmly for five seconds. The participants then recorded the number of days before the first artificial nail and the second artificial nail lost complete adhesion to the natural nail and fell or 'popped' off.

In addition, a traditional nail adhesive formulation containing Ethyl cyanoacrylate, Polymethyl methacrylate and the phenolic inhibitor Butylated Hydroxy Anisole (BHA) was used to apply artificial nails to the natural nail of the less dominant hand by the method described above. Similarly, the participants then recorded the number of days before the first artificial nail and the second artificial nail lost complete adhesion to the natural nail and fell or 'popped' off.

The formulation of the traditional nail adhesive is as provided in Table 2 below:

**Table 2**

| **Formulation 2** | **CAS#** | **Percent w/w** |
|---|---|---|
| Ethyl cyanoacrylate | 7085-85-0 | 95.3 |
| Polymethyl methacrylate (PMMA) | 9011-14-7 | 4.5 |
| Butylated Hydroxy Anisole (BHA) | 25013-16-5 | 0.2 |
| **TOTAL** | | **100** |

The results of the test are provided in Table 3 below.

**Table 3**

| **Participant** | **Pop Off First Nail (Days)** | |
|---|---|---|
| | **Formulation 1** | **Formulation 2** |
| 1 | 8 | 6 |
| 2 | 10 | 6 |
| 3 | 1 | 1 |
| 4 | 7 | 4 |
| 5 | 10 | 7 |
| 6 | 8 | 6 |
| 7 | 6 | 6 |
| 8 | 4 | 3 |
| 9 | 3 | 3 |
| 10 | 7 | 8 |
| 11 | 5 | 2 |
| 12 | 6 | 7 |
| 13 | 9 | 8 |
| 14 | 8 | 9 |
| 15 | 8 | 5 |
| 16 | 10 | 7 |
| 17 | 5 | 2 |
| 18 | 5 | 3 |
| 19 | 8 | 4 |
| 20 | 9 | 7 |
| **Total** | **137** | **104** |
| | | |
| **Mean** | **6.85** | **5.20** |
| **Median** | **7.5** | **6** |
| **Increase** - **Mean** | **32%** | - |
| **Increase** - **Median** | **25%** | - |

The increase in the mean and the median days for the longevity of the artificial nail adhered to the natural nail between Formulation 1 and Formulation 2 is 32% and 25% respectively.

**Table 4**

| **Participant** | **Pop Off Second Nail (Days)** | |
|---|---|---|
| | **Formulation 1** | **Formulation 2** |
| 1 | 9 | 7 |
| 2 | 12 | 8 |
| 3 | 3 | 2 |
| 4 | 8 | 11 |
| 5 | 12 | 11 |
| 6 | 10 | 7 |
| 7 | 9 | 7 |
| 8 | 6 | 5 |
| 9 | 4 | 4 |
| 10 | 8 | 8 |
| 11 | 5 | 3 |
| 12 | 7 | 8 |
| 13 | 12 | 9 |
| 14 | 9 | 9 |
| 15 | 10 | 7 |
| 16 | 11 | 9 |
| 17 | 8 | 4 |
| 18 | 6 | 4 |
| 19 | 11 | 7 |
| 20 | 10 | 8 |
| **Total** | **170** | **138** |
| | | |
| **Mean** | **8.5** | **6.9** |
| **Median** | **9** | **7** |
| **Increase** - **Mean** | **23%** | - |
| **Increase** - **Median** | **29%** | - |

The increase in the mean and the median days for the longevity of the artificial nail adhered to the natural nail between Formulation 1 and Formulation 2 is 23% and 29% respectively.

Formulation 1 was applied to the dominant hand and the nails would generally be exposed to more wear than the less dominant hand. This confirms that the substitution of BHA inhibitor by *α*-tocopherol in the nail adhesive formulation improves the adhesion of the artificial nail to the natural nail by a statistically significant amount.

### Test results - tensile

It is not possible to devise an experiment by which the lap shear tensile strength of an artificial nail bonded to the natural nail by use of a nail adhesive can easily be measured. An acceptable substitute test is to measure the lap shear strength of Formulation 1 compared to Formulation 2 by the following method:
1) Lightly abrade the ends of a mild steel strip measuring 100mm x 25mm x 1.5mm to smooth off the uneven edges.
2) Thoroughly clean the ends of the mild steel strips with acetone to remove any grease or dirt.
3) Apply a small amount of the adhesive to the cleaned end of the steel strip. Position the second steel strip on top the first steel strip to create a 12.5mm overlap.
4) Hold the steel strips together using bulldog clips and leave to cure at 20°C for 24 hours then clamp the test piece in the jaws of the tensile tester and set the machine in motion (1mm/min).
5) Record the results when the test piece breaks. Repeat a further two times.

The above lap shear tensile strength test was repeated exactly as described above but with two strips of acrylonitrile butadiene styrene (ABS), a common thermoplastic polymer.

**Table 5**

| **Lap Shear Strength N/mm² Mild Steel** | |
|---|---|
| **Formulation 1** | **Formulation 2** |
| 15.9 | 12.3 |
| 16.4 | 11.6 |
| 16.1 | 12.2 |
| Mean **16.1** | Mean **12.0** |

The mean lap shear tensile strength measured for Formulation 1 was 16.1 N/mm² and for Formulation 2, the mean lap shear strength was measured at 12.0 N/mm². There is a 34% increase in lap shear tensile strength for Formulation 1 compared to Formulation 2. This confirms that the substitution of BHA inhibitor by *α*-tocopherol in the nail adhesive formulation improves the lap shear strength by a statistically significant amount when tested on mild steel strips.

**Table 6**

| **Lap hear Strength N/mm² ABS** | |
|---|---|
| **Formulation 1** | **Formulation 2** |
| 4.1 | 3.1 |
| 3.8 | 2.6 |
| 3.6 | 2.9 |
| Mean **3.8** | Mean **2.9** |

The mean lap shear tensile strength measured for Formulation 1 was 3.8 N/mm² and for Formulation 2, the mean lap shear strength was measured at 2.9 N/mm². There is a 31% increase in lap shear tensile strength for Formulation 1 compared to Formulation 2. This confirms that the substitution of BHA inhibitor by *α*-tocopherol in the nail adhesive formulation improves the lap shear strength by a statistically significant amount when tested on ABS strips.

It should be understood that the embodiments described herein are merely exemplary and that a person skilled in the art may make many variations and modifications without departing from the scope of the invention as defined by the claims.

### Variations

The above embodiments utilise the *α*-tocopherol Vitamin E compound. Other Vitamin E compounds are also possible, specifically *β*-tocopherol, *γ*-tocopherol, *δ-*tocopherol, *α*-tocotrienol, *β*-tocotrienol, *γ*-tocotrienol and *δ*-tocotrienol.

In an embodiment, the antioxidant inhibitor is *β*-tocopherol , which is present in an amount from 0.02wt% to 0.5wt% of the composition.

In an embodiment, the antioxidant inhibitor is *γ*-tocopherol, which is present in an amount from about 0.02 wt % to 0.5 wt % of the composition.

In an embodiment, the antioxidant inhibitor is *δ*-tocopherol, which is present in an amount from about 0.02 wt % to 0.5 wt % of the composition.

In an embodiment, the antioxidant inhibitor is *α*-tocotrienol, which is present in an amount from about 0.02 wt % to 0.5 wt % of the composition.

In an embodiment, the antioxidant inhibitor is *β*-tocotrienol, which is present in an amount from about 0.02 wt % to 0.5 wt % of the composition.

In an embodiment, the antioxidant inhibitor is *γ*-tocotrienol, which is present in an amount from about 0.02 wt % to 0.5 wt % of the composition.

In an embodiment, the antioxidant inhibitor is *δ* -tocotrienol, which is present in an amount from about 0.02 wt % to 0.5 wt % of the composition.

In an embodiment, the thickener is PMMA.

In an embodiment, the thickener is PMMA in combination with fumed silica.

In addition to the embodiments above, a plasticiser may be added.

In an embodiment, the plasticiser is Triacetin which is present in an amount from 2wt% to 15wt%.

In an embodiment, the plasticiser is Tributyl Citrate which is present in an amount from 2wt% to 15wt%.

In addition to the embodiments above, a colourant may be added.

In an embodiment, the colourant is D&C Red 7 which is present in an amount from 0.001wt% to 0.005wt%.

## Claims

1. A nail glue composition comprising:
at least one cyanoacrylate of the formula:
CH₂=C(CN)COOR
wherein R is ethyl, methoxyethyl, ethoxyethyl, n-butyl or octyl or mixtures thereof;
an anionic stabiliser; and
an antioxidant stabiliser comprising at least one stabiliser compound selected from the group consisting of:
*α*-tocopherol, *β*-tocopherol, *γ*-tocopherol, *δ*-tocopherol, *α*-tocotrienol, *β-*tocotrienol, *γ*-tocotrienol and *δ*-tocotrienol; and
derivatives thereof;
in which the at least one stabiliser compound is in an amount of at least 0.02wt% of the nail glue composition.

2. A nail glue composition according to claim 1, wherein the at least one stabiliser compound is present in an amount of at least 0.1wt% of the nail glue composition.

3. A nail glue composition according to claim 2, wherein the at least one stabiliser compound is present in an amount of at least 0.2wt% of the nail glue composition.

4. A nail glue composition according to any preceding claim, wherein the at least one cyanoacrylate is in the range of 85 to 99 wt % of the nail glue composition.

5. A nail glue composition according to any preceding claim, wherein the at least one stabiliser compound is selected from the group consisting of:
*α*-tocopherol, *β*-tocopherol, *γ*-tocopherol, *δ*-tocopherol, *α*-tocotrienol, *β*-tocotrienol, *γ*-tocotrienol and *δ*-tocotrienol.

6. A nail glue composition according to any preceding claim, wherein the antioxidant stabiliser comprises at least 0.02wt% *α*-tocopherol.

7. A nail glue composition according to any preceding claim, comprising a thickener.

8. A nail glue composition according to claim 7, wherein the thickener is polymethyl methacrylate.

9. A nail glue composition according to claim 8, wherein the polymethyl methacrylate is present in the range of 0.1wt% to 14wt% of the nail glue composition.

10. A nail composition according to any preceding claim, wherein the antioxidant stabiliser is present in the range 0.03% to 0.7% of the nail glue composition.

11. A nail composition according to any preceding claim, comprising a plasticiser.

12. A nail glue composition according to claim 11, wherein the plasticiser is selected from Triacetin and Tributyl Citrate.

13. A nail glue composition according to claim 11 or 12, wherein the plasticiser is present in the range 2wt% to 15wt%.

14. A nail glue composition according to any preceding claim, comprising a colourant.

15. A nail glue composition according to claim 14, wherein the colourant is D&C Red 7.

16. A nail glue composition according to claim 14 or 15, wherein the colourant is present in the range 0.001wt% to 0.005wt%
